# EUROPEAN PATENT SPECIFICATION

(11) **EP 0 572 506 B1**
(45) Date of publication and mention of the grant of the patent: **08.01.1997**
(21) Application number: 92905989.7
(22) Date of filing: 13.02.1992
(51) Int. Cl.: A61H 7/00, A61N 2/08

(54) **MASSAGING DEVICE**
Massagegerät
DISPOSITIF DE MASSAGE

(30) Priority: 18.02.1991 NL 9100277
(43) Date of publication of application: 08.12.1993
(73) Proprietor: JACOBI, Jacob, NL-4251 KB Werkendam (NL)
(72) Inventor: JACOBI, Jacob, NL-4251 KB Werkendam (NL)
(74) Representative: Lips, Hendrik Jan George, Ir.
(86) International application number: NL9200032
(87) International publication number: WO9214435

(56) References cited:
- WO-A-86/03664
- WO-A-86/04809
- FR-A- 2 341 285
- FR-A- 2 592 579
- US-A- 2 795 224
- US-A- 4 082 089
- US-A- 4 691 693
- SOVIET PATENTS ABSTRACTS Section PQ, Week 8818, 5 May 1988 Derwent Publications Ltd., London, GB; Class P33, AN 88-125035/18 & SU,A,1 342 501 (GLAVMOSPROMSTROIMAT) 7 October 1987

## Description

The invention relates to a massaging device comprising a handle and a massaging section, a heating element being present in said handle for heating the massaging section, said massaging section comprising means for generating a magnetic field around it.

Such a massaging device is known from US-A-4 691 693. In case of this known device the massaging section is mounted on a pin extending from the handle. Said pin is heated by the heating element provided in said handle.

Now the object of the invention is to improve the known device and that in particular the device will offer more possibillities and can be used more effectively.

According to the invention this object is reached by applying the features as indicated in the characterizing portion of claim 1.

According to a further elaboration the convex part of said spoon shaped massaging section, at least partly, can be furnished with a bumpy surface.

In WO86/04809 a massaging device is described provided with a fan by which air can be passed along a heating element present in the handle of the device towards the massaging section. This device, however, is suitable for vibratory massage therapy and is neither provided with magnetic means nor with a spoon shaped massaging section.

The embodiment according to fig. 10 of said publication shows a massaging device comprising a massage head provided with openings and secured to spacer posts so that an annular vent is defined.

It has appeared that the effect of the north pole of the magnetic field differs from that of the south pole. The north pole can be effectively used for fighting inflammations, infections, virus and such like and the south pole can be effectively used for fighting chronic pains and such like.

The treatment with the magnetic field may well be used in conjunction with treatment by heating, infra-red rays, sound waves, vibration, laser beams and other techniques used for these purposes.

The magnetic field can be generated both by a permanent magnet and an electromagnet. In the latter case the field can be interrupted by means of a switch provided in the handle.

Now the invention will be described more in detail by 20 reference to the accompanying drawings, in which:
Fig. 1 schematically shows an embodiment, partly in plan view and partly in sectional view, of a massaging device according to the invention and
Fig. 2 and 3 show a plan view and a side view resp. of the massaging section of the device shown in fig. 1.

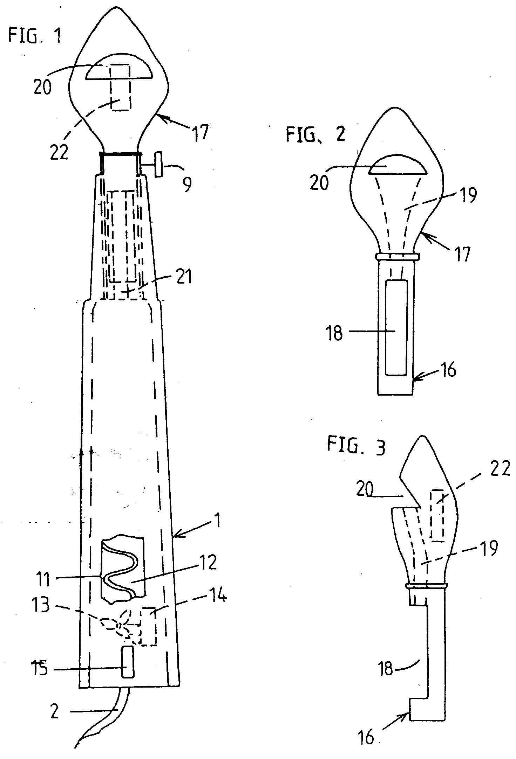

Fig. 1 shows a massaging device comprising a handle 1 with a current supply wire 2 for supplying current.

In the handle 1, a heating element 11 is provided within a channel 12 through which air can be passed by means of a fan 13 driven by a small motor 14. A switch 15 is present for supplying current from the supply wire 2 to the heating element 11 and to the motor 14 of the fan 13.

At the other end the channel 12 is shaped such that the shaft 16 of a massaging section 17 can be inserted therein. In the shaft 16, an opening 18 is present as appears in particular from the figures 2 and 3. The opening 18 is connected to an air passage 19 in the massaging section 17, said passage debouching into an opening 20 in the back side of said section. In this way, the massaging section 17 can be heated by means of the air flowing through the channels 12 and 19 towards the opening 20.

In the wall of the channel 12 opposite the shaft 16, a groove 21 is provided for passing the air from the channel 12 into the opening 18. By rotating the massaging section 17 in respect of the handle 1 the opening 18 can be brought more or less in overlapping relation with the groove 21 and by this, the amount of air flowing towards the massaging section 17 and out of the opening 20 can be regulated. After adjusting the massaging section 17 it can be fixed by means of a set screw 9.

As appears in particular from fig. 3, in the massaging section 17 a permanent magnet 22 is positioned. Instead of a permanent magnet, an electromagnet can also be used which can be energized by a current supply and a switch provided in the handle 1 in a way not further indicated.

It is also possible to use a metal massaging section which is previously magnetized. A disadvantage of doing so is the fact that it will be demagnetized in the long run, in particular because the section is heated.

## Claims

1. A massaging device comprising a handle (1) and a massaging section (17), a heating element (11) being present in said handle for heating said massaging section, said massaging section (17) comprising means (22) for generating a magnetic field around it, **characterized in that** in said handle (1) a channel (12) is provided through which, by means of a fan (13), air can be passed along the heating element (11) present in said handle and towards said massaging section (17) which is having the shape of a spoon, one surface of it being provided with a hollow section and with an air inlet opening (19) connected to the channel (12) in said handle (1) and with an air outlet opening (20) positioned at distance from the handle (1) so that a flow of heated air can pass through a part of said spoon shaped massaging section (17) for heating this.

2. A massaging device according to claim 1, **characterized in** that the convex part of said spoon shaped massaging section (17) is at least partly furnished with a bumpy surface.

## Patentansprüche

1. Massagegerät mit einem Handgriff (1) und einem Massageabschnitt (17), einem Heizelement (11), das zum Erhitzen des Massageabschnittes in dem Handgriff vorhanden ist, wobei der Massageabschnitt (18) Einrichtungen (22) zur Erzeugung eines Magnetfeldes um ihn herum umfaßt, **dadurch gekennzeichnet**, daß in dem Handgriff (1) ein Kanal (12) vorgesehen ist, durch welchen mit Hilfe eines Ventilators (13) Luft entlang dem Heizelement (11) in dem Handgriff und zu dem Massageabschnitt (11) geführt werden kann, welcher die Form eines Löffels hat, von dem eine Oberfläche mit einem hohen Abschnitt und mit einer Lufteinlaßöffnung (19) versehen ist, die mit dem Kanal (12) in dem Handgriff (1) und mit einer Luftauslaßöffnung (20), die in einem Abstand von dem Handgriff (1) derart angeordnet ist, daß eine Strom von erhitzter Luft durch einen Teil des löffelförmigen Massageabschnittes (17) zum Erhitzen desselben gehen kann, verbunden ist.

2. Massagegerät nach Anspruch 1, **dadurch gekennzeichnet,** daß der konvexe Teil des löffelförmigen Massageabschnittes (17) wenigstens teilweise mit einer zerfurchten Oberfläche ausgestattet ist.

## Revendications

1. Dispositif de massage comprenant un manche (1) et une section de massage (17), un élément chauffant (11) étant présent dans ledit manche pour chauffer ladite section de massage, ladite section de massage (17) comprenant des moyens (22) pour produire un champ magnétique autour d'elle, caractérisé en ce qu'il est prévu dans ledit manche (1) un canal (12) à travers lequel un ventilateur (13) peut faire passer de l'air le long de l'élément chauffant (11) contenu dans ledit manche et en direction de ladite section de massage (17) qui a la forme d'une cuiller, dont une surface présente une section creuse et une ouverture d'arrivée d'air (19) raccordée au canal (12) dans ledit manche (12) et une ouverture de sortie d'air (20) située à distance du manche (1) de sorte qu'un courant d'air chaud peut passer à travers une partie de ladite section de massage en forme de cuiller (17) pour la chauffer.

2. Dispositif de massage selon la revendication 1, caractérisé en ce que la partie convexe de ladite section de massage en forme de cuiller (17) présente au moins en partie une surface bosselée.
